(19) Europäisches Patentamt
European Patent Office
Office européen des brevets

(11) **EP 4 317 195 A1**

(12) **EUROPEAN PATENT APPLICATION**
published in accordance with Art. 153(4) EPC

(43) Date of publication:
**07.02.2024 Bulletin 2024/06**

(21) Application number: **22780889.6**

(22) Date of filing: **29.03.2022**

(51) International Patent Classification (IPC):
**C08F 2/00** (2006.01)     **A61F 13/53** (2006.01)
**B01J 20/26** (2006.01)     **C08F 120/06** (2006.01)

(52) Cooperative Patent Classification (CPC):
**A61F 13/53; B01J 20/26; C08F 2/00; C08F 120/06**

(86) International application number:
**PCT/JP2022/015354**

(87) International publication number:
**WO 2022/210678 (06.10.2022 Gazette 2022/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.03.2021   JP 2021057723**

(71) Applicant: **Sumitomo Seika Chemicals Co., Ltd.**
**Kako-gun, Hyogo 675-0145 (JP)**

(72) Inventor: **KITANAKA, Hironobu**
**Himeji-shi, Hyogo 672-8076 (JP)**

(74) Representative: **Mewburn Ellis LLP**
**Aurora Building**
**Counterslip**
**Bristol BS1 6BX (GB)**

(54) **WATER-ABSORBENT RESIN, ABSORBENT BODY AND ABSORBENT ARTICLE**

(57)     Provided is a water-absorbent resin capable of inhibiting backflow and leakage of a liquid absorbed by an absorbent body. When measured by the following measurement method, this water-absorbent resin shows a repeated water absorption amount in an inclined state of 35 g or more. <Method for measuring repeated water absorption amount in inclined state> 1.0 g of the water-absorbent resin is uniformly spread in a circular plastic petri dish having an inner diameter of 8.5 cm and a height of 1.7 cm. Subsequently, 30 g of physiological saline is supplied at once on the water-absorbent resin. Immediately thereafter, the petri dish is shaken 10 times from side to side and then allowed to stand for 3 minutes to form a swollen gel layer in which the physiological saline is absorbed by the water-absorbent resin. Separately, a device is prepared by fixing an acrylic plate having a flat main surface in such a manner that the main surface is at an angle of 30° to the horizontal plane. On the upper surface of the swollen gel layer, an air-through non-woven fabric, which has a weight per unit area of 21 g/m2 and cut into the same size as the inside of the petri dish, is placed. Then, the petri dish is stuck to the acrylic plate with tape to avoid slipping off. From a vertical height of approximately 1 cm at the center of the swollen gel layer in the petri dish, 5 g of the physiological saline is poured for 5 seconds using a macropipette. The pouring procedure is repeated 8 times at 1 minute intervals from the start of the physiological saline pouring. The amount of the liquid leaked from the petri dish is measured and subtracted from the amount (i.e., 40 g) of the poured physiological saline. The thus calculated mass of the physiological saline that is retained by the swollen gel layer is regarded as the repeated water absorption amount of the water-absorbent resin.

FIG. 1

**Description**

TECHNICAL FIELD

The present invention relates to a water-absorbent resin, an absorbent body, and an absorbent article and more particularly to a water-absorbent resin constituting an absorbent body suitably used for sanitary supplies such as disposable diapers, sanitary napkins, incontinence pads, an absorbent body employing the water-absorbent resin, and an absorbent article.

BACKGROUND ART

In recent years, water-absorbent resins have been widely used in the field of sanitary supplies such as disposable diapers, sanitary napkins, and incontinence pads.

As such a water-absorbent resin, a crosslinked product of a polymer of water-soluble ethylenically unsaturated monomers, specifically a crosslinked product of a partially neutralized acrylic acid polymer, is considered to be a preferable water-absorbent resin because it has a better water-absorbing capacity, and acrylic acid as a raw material thereof is easily industrially available, which has many advantages such as an ability to be produced at a low cost with a uniform quality and little likelihood of being putrefied or deteriorated (see, for example, Patent Document 1).

On the other hand, an absorbent article such as a disposable diaper, a sanitary napkin, and an incontinence pad mainly includes an absorbent body that absorbs and holds body fluids such as urine and menstrual blood excreted from the body and is disposed in a central portion, a liquid-permeable surface sheet (top sheet) disposed on a side in contact with the body, and a liquid-impermeable underside sheet (back sheet) disposed on a side opposite to the side in contact with the body. The absorbent body is usually composed of hydrophilic fibers such as pulp and a water-absorbent resin.

PRIOR ART DOCUMENT

PATENT DOCUMENT

Patent Document 1: Japanese Patent Laid-open Publication No. H3-227301

SUMMARY OF THE INVENTION

PROBLEMS TO BE SOLVED BY THE INVENTION

In such an absorbent article, the water-absorbent resin contained in the absorbent body is required to have a high water retention. By increasing the water retention of the water-absorbent resin, it is possible to ameliorate a phenomenon in which backflow of the liquid once absorbed in the absorbent body occurs (that is, it is a phenomenon in which the liquid returns from the absorbent body, and when the absorbent body is touched with a hand, uncomfortable wetting is felt). However, in the case where the absorbent surface of the absorbent article is inclined at the time of use of the absorbent article, the repeatedly excreted liquid is not sufficiently absorbed, and there may be a problem that the liquid leaks out of the absorbent article although the water retention of the water-absorbent resin is high.

Under such circumstances, a main object of the present invention is to provide a water-absorbent resin capable of suppressing backflow and leakage of liquid absorbed in an absorbent body.

MEANS FOR SOLVING THE PROBLEM

The present inventor has conducted intensive studies in order to solve the above problems. As a result, the present inventor have found that when a water-absorbent resin having a repeated water absorption amount in an inclined state of 35 g or more as measured by a predetermined method is applied to an absorbent body, backflow and leakage of liquid can be suitably suppressed. The present invention has been completed through further intensive studies based on such findings.

That is, the present invention provides an invention having the following configurations.

Item 1. A water-absorbent resin having a repeated water absorption amount in an inclined state of 35 g or more as measured by a measurement method below.

<Method for Measuring Repeated Water Absorption Amount in Inclined State>

In a petri dish made of plastic and having a circular shape, an inner diameter of 8.5 cm, and a height of 1.7 cm, 1.0 g of the water-absorbent resin is uniformly spread. Subsequently, 30 g of physiological saline is supplied at once on the water-absorbent resin, and immediately the petri dish is shaken 10 times from side to side and then allowed to stand for 3 minutes to form a swollen gel layer containing the water-absorbent resin having absorbed the physiological saline. Separately, a device is prepared including an acrylic plate having a main surface having a flat shape fixed in such a manner that the main surface is at an angle of 30° to the horizontal plane. On an upper surface of the swollen gel layer, an air through non-woven fabric having a weight per unit area of 21 $g/m^2$ and cut into the same size as the inside of the petri dish is placed, and the petri dish is fixed to the acrylic plate with tape to avoid slipping off. From a vertical height of approximately 1 cm at the center of the swollen gel layer in the petri dish, 5 g of the physiological saline is poured for 5 seconds using a macropipette. The pouring is repeated eight times at one minute intervals from the start of the pouring of the physiological saline. The amount of liquid leaked from the petri dish is measured and subtracted from the poured amount of 40 g of the physiological saline, and the calculated mass of the physiological saline retained by the swollen gel layer is regarded as the repeated water absorption amount of the water-absorbent resin.

Item 2. The water-absorbent resin according to item 1, having a median particle diameter of 200 $\mu$m or more and 700 $\mu$m or less.

Item 3. The water-absorbent resin according to item 1 or 2, having a water absorption speed of the physiological saline of 20 seconds or more and 70 seconds or less.

Item 4. An absorbent body including the water-absorbent resin according to any one of items 1 to 3.

Item 5. An absorbent article including the absorbent body according to item 4.

ADVANTAGES OF THE INVENTION

The present invention can provide a water-absorbent resin capable of suppressing backflow and leakage of liquid absorbed in an absorbent body. Furthermore, the present invention can also provide an absorbent body and an absorbent article including the water-absorbent resin.

BRIEF DESCRIPTION OF THE DRAWINGS

Fig. 1 schematically shows a measuring device used for measuring a non-pressurized demand wettability (DW) of a water-absorbent resin.
Fig. 2 is a schematic diagram for illustrating a method of a leakage property test of an absorbent article.

EMBODIMENT OF THE INVENTION

In the present specification, the term "comprising" includes "consisting essentially of" and "consisting of". In addition, in the present specification, "(meth)acrylic" means "acrylic or methacrylic", and "(meth)acrylate" means "acrylate or methacrylate".

In addition, in the present specification, numerical values joined by "to" means a numerical range including numerical values before and after "to" as a lower limit value and an upper limit value. When a plurality of lower limit values and a plurality of upper limit values are described separately, an arbitrary lower limit value and an arbitrary upper limit value can be selected and connected by "to".

1. Water-Absorbent Resin

A water-absorbent resin of the present invention is characterized in that the repeated water absorption amount in an inclined state is 35 g or more as measured by a measurement method below. The water-absorbent resin of the present invention having such a characteristic can suppress backflow and leakage of liquid absorbed in an absorbent body. Hereinafter, the water-absorbent resin of the present invention will be described in detail.

<Method for Measuring Repeated Water Absorption Amount in Inclined State>

In a petri dish made of plastic and having a circular shape, an inner diameter of 8.5 cm, and a height of 1.7 cm, 1.0 g of the water-absorbent resin is uniformly spread. Subsequently, 30 g of physiological saline is supplied at once on the water-absorbent resin, and immediately the petri dish is shaken 10 times from side to side and then allowed to stand for 3 minutes to form a swollen gel layer containing the water-absorbent resin having absorbed the physiological saline. Separately, a device is prepared including an acrylic plate having a main surface having a flat shape fixed in such a manner that the main surface is at an angle of 30° to the horizontal plane. On an upper surface of the swollen gel layer, an air through non-woven fabric having a weight per unit area of 21 g/mand cut into the same size as the inside of the petri dish is placed, and the petri dish is fixed to the acrylic plate with tape to avoid slipping off. From a vertical height of approximately 1 cm at the center of the swollen gel layer in the petri dish, 5 g of the physiological saline is poured for 5 seconds using a macropipette. The pouring is repeated eight times at one minute intervals from the start of the pouring of the physiological saline. The amount of liquid leaked from the petri dish is measured and subtracted from the poured amount of 40 g of the physiological saline, and the calculated mass of the physiological saline retained by the swollen gel layer is regarded as the repeated water absorption amount of the water-absorbent resin. A more specific measurement method is as described in the EXAMPLES section.

From the viewpoint of more suitably exerting the effect of the present invention, the repeated water absorption amount in an inclined state of the water-absorbent resin of the present invention is preferably 36 g or more, more preferably 37 g or more, still more preferably 38 g or more, still more preferably 39 g or more, most preferably 40 g (maximum value).

Further, from the viewpoint of more suitably exerting the effect of the present invention, the physiological saline retention of the water-absorbent resin of the present invention may be 40 g/g or more, 42 g/g or more, 44 g/g or more, 46 g/g or more, 48 g/g or more, 50 g/g or more, 53 g/g or more, or 60 g/g or more, and the retention may be 80 g/g or less, 75 g/g, 70 g/g or less, 65 g/g or less, 63 g/g or less, 60 g/g or less, 55 g/g or less, or 52 g/g or less. A method for measuring the physiological saline retention is as follows.

<Method for Measuring Physiological Saline Retention>

A cotton bag (No. 60 count cotton broadcloth, 100 mm in width × 200 mm in length) in which 2.0 g of the water-absorbent resin has been weighed out is placed in a 500-mL beaker. Into the cotton bag containing the water-absorbent resin, 500 g of a 0.9-mass% aqueous sodium chloride solution (physiological saline) is poured at once so as not to form lumps, the upper portion of the cotton bag is tied with a rubber band, and the cotton bag is left to stand for 30 minutes to allow the water-absorbent resin to swell. The cotton bag after 30 minutes is dehydrated for 1 minute using a dehydrator set so that the centrifugal force is 167 G, and the mass Wa (g) of the cotton bag containing the swollen gel after dehydration is measured. The same operation is performed without adding the water-absorbent resin, the empty mass Wb (g) of the cotton bag in a wet state is measured, and the physiological saline retention is calculated from the following equation.

$$\text{Physiological saline retention (g/g)} = [Wa - Wb]/2.0$$

From the viewpoint of more suitably exerting the effect of the present invention, the 5-minute non-pressurized DW (Demand Wettability) of the water-absorbent resin of the present invention may be 36 ml/g or more, 40 ml/g or more, 45 ml/g or more, 48 ml/g or more, 50 ml/g or more, 52 ml/g or more, 54 ml/g or more, 55 ml/g or more, or 60 ml/g or more and may be 90 ml/g or less, 80 ml/g or less, 75 ml/g or less, 70 ml/g or less, 60 ml/g or less, 55 ml/g or less, or 50 ml/g or less. A more specific measurement method is as described in the EXAMPLES section.

<Measurement of Five-Minute Non-Pressurized DW (Demand Wettability)>

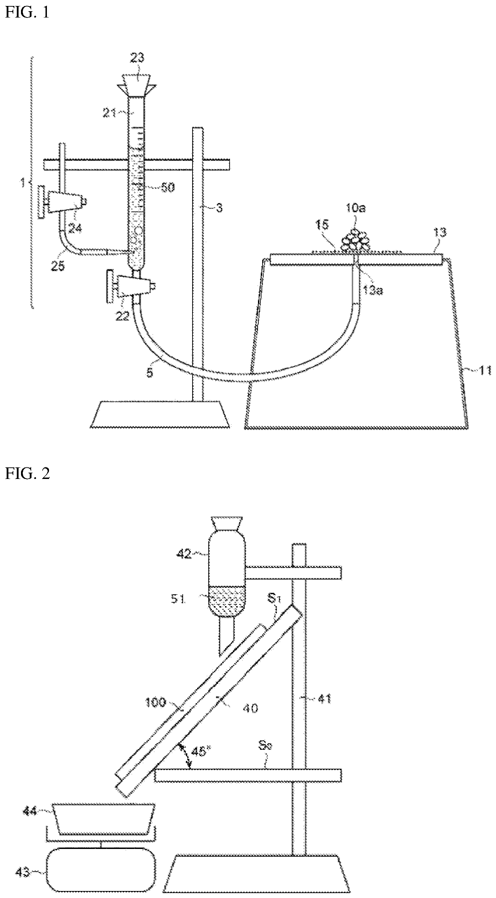

The non-pressurized DW of particles of the water-absorbent resin is measured using a measuring device shown in the schematic diagram of Fig. 1. The measurement is performed five times for one kind of water-absorbent resin, and the average value of the measured values of three points excluding the minimum value and the maximum value is determined. The measuring device includes a burette unit 1, a conduit 5, a measurement stage 13, a nylon mesh sheet 15, a stand 11, and a clamp 3. The burette unit 1 includes a graduated burette tube 21, a rubber stopper 23 for tightly sealing an upper opening of the burette tube 21, a cock 22 connected to a lower tip of the burette tube 21, and an air introduction tube 25 and a cock 24 connected to a lower portion of the burette tube 21. The burette unit 1 is fixed with the clamp 3. The flat plate-shaped measurement stage 13 has a through hole 13a having a diameter of 2 mm formed in a central portion thereof and is supported by the height-adjustable stand 11. The through hole 13a of the measurement stage 13 and the cock 22 of the burette unit 1 are connected by the conduit 5. The inner diameter of the conduit 5 is 6 mm.

First, the cock 22 and the cock 24 of the burette unit 1 are closed, and 0.9-mass% saline 50 adjusted to 25°C is put into the burette tube 21 from the opening of the upper portion of the burette tube 21. The concentration of the saline of 0.9 mass% is a concentration based on the mass of the saline. After the opening of the burette tube 21 was tightly sealed with the rubber stopper 23, the cock 22 and the cock 24 were opened. The inside of the conduit 5 is filled with the 0.9-mass% saline 50 so that air bubbles do not enter. The height of the measurement stage 13 is adjusted such that the height of the surface of the 0.9-mass% saline reaching the inside of the through hole 13a becomes equal to the height of the upper surface of the measurement stage 13. After the adjustment, the height of the surface of the 0.9-mass% saline 50 in the burette tube 21 is read using the graduations on the burette tube 21, and the position is set as a 0 point (reading at 0 seconds).

The nylon mesh sheet 15 (100 mm × 100 mm, 250 mesh, thickness of about 50 μm) is laid in the vicinity of the through hole 13a on the measurement stage 13, and a cylinder having an inner diameter of 30 mm and a height of 20 mm is placed at the center thereof. In the cylinder, 1.00 g of a water-absorbent resin 10a is uniformly spread. Thereafter, the cylinder is carefully removed to obtain a sample in which the water-absorbent resin 10a is circularly dispersed on the central portion of the nylon mesh sheet 15. Next, the nylon mesh sheet 15 on which the water-absorbent resin 10a is placed is moved quickly to such an extent that the water-absorbent resin 10a is not scattered so that the center of the nylon mesh sheet 15 is located at the position of the through hole 13a, and measurement is started. A point of time when air bubbles are first introduced from the air introduction tube 25 into the burette tube 21 is defined as the start of water absorption (0 seconds).

A decrease amount (that is, the amount of the 0.9-mass% saline absorbed by the water-absorbent resin 10a) of the 0.9-mass% saline 50 in the burette tube 21 was sequentially read, and a decrement Wc (g) of the 0.9-mass% saline 50 after 5 minutes from the start of water absorption by the water-absorbent resin 10a was read. From the decrement Wc, the five-minute non-pressurized DW is obtained by the following equation. The non-pressurized DW is a water absorption amount per 1.00 g of the water-absorbent resin 10a.

$$\text{Non-pressurized DW value (mL/g)} = \text{Wc}/1.00$$

From the viewpoint of more suitably exerting the effect of the present invention, the water absorption speed of the physiological saline of the water-absorbent resin of the present invention may be 20 seconds or more, 30 seconds or more, 33 seconds or more, 35 seconds or more, 38 seconds or more, 42 seconds or more, or 45 seconds or more and may be 70 seconds or less, 60 seconds or less, 50 seconds or less, or 45 seconds or less. A method for measuring the physiological saline absorption rate is as follows.

<Method for Measuring Physiological Saline Absorption Rate>

The physiological saline absorption rate of the water-absorbent resin is measured by the following procedure based on the vortex method. First, 50 ± 0.1 g of physiological saline adjusted to a temperature of 25 ± 0.2°C in a constant temperature water bath is weighed out in a beaker having an internal volume of 100 mL. Next, the physiological saline is stirred at a rotation speed of 600 rpm using a magnetic stir bar (8 mmφ × 30 mm, without ring) to generate a vortex. To the physiological saline, 2.0 ± 0.002 g of the water-absorbent resin is added at once. The time [sec] from the addition of the water-absorbent resin to the time point at which the vortex on the liquid surface converges is measured, and the time is taken as the water absorption speed of the water-absorbent resin.

As for the water-absorbent resin of the present invention, the number of times of input of the liquid until occurrence of leakage measured by the following leakage test is preferably four or more. The number of times of input is, for example, seven times or less. The absorption amount of the liquid until the occurrence of leakage is preferably 240 g or more, more preferably 270 g or more. The absorption amount of the liquid until the occurrence of leakage is, for example, 550 g or less, 520 g or less, or 490 g or less.

<Leakage Test (Gradient Absorption Test)>

As illustrated in the schematic diagram of Fig. 2, a supporting plate 40 (an acrylic resin plate in this case, hereinafter also referred to as an inclined surface $S_1$) having a flat main surface and a length of 45 cm is fixed by a stand 41 in a state of being inclined at 45 ± 1° with respect to a horizontal plane $S_0$. A test absorbent article 100 (15.1 g) is stuck on the inclined surface $S_1$ of the fixed supporting plate 40 in such an orientation that the longitudinal direction thereof is along the longitudinal direction of the supporting plate 40. Next, a test liquid 51 (described later) adjusted to 25 ± 1°C is dropped from a dropping funnel 42 disposed vertically above the absorbent article toward a position 8 cm above the center of the absorbent body in the absorbent article 100. At a rate of 8 mL/sec, 80 mL per one time of the test liquid is added dropwise. The distance between the tip of the dropping funnel 42 and the absorbent article is 10 ± 1 mm. The test liquid is repeatedly input under the same conditions at intervals of 10 minutes from the start of the first input of the test liquid. The test liquid is input until leakage is observed, and the number of times of input is defined as the number of times of input until occurrence of leakage.

When the test liquid that has not been absorbed by the absorbent article 100 leaks from the lower portion of the supporting plate 40, the leaked test liquid is collected in a metal tray 44 disposed below the supporting plate 40. The mass (g) of the collected test liquid is measured with a balance 43, and the value is recorded as the leakage amount. The absorption amount until leakage occurs is calculated by subtracting the leakage amount from the total input amount of the test liquid. As this numerical value is larger, leakage of liquid is judged to be less likely to occur when the absorbent article is worn. The density of the test liquid was supposed to be 1.0 g/mL.

<Preparation of Test Liquid>

The test liquid is prepared by blending and dissolving an inorganic salt in ion-exchanged water so that the inorganic salt is present as described below and further blending a small amount of Brilliant Blue FCF.

Composition of test liquid

- Deionized water: 5919.6 g
- NaCl: 60.0 g
- $CaCl_2 \cdot H_2O$: 1.8 g
- $MgCl_2 \cdot 6H_2O$: 3.6 g
- Brilliant Blue FCF for food (for coloring)
- 1% Triton X-100: 15.0 g

As for the water-absorbent resin of the present invention, the first backflow amount measured by the following backflow test is preferably 15 g or less, more preferably 12 g or less. In addition, the second backflow amount is preferably 72 g or less, more preferably 70 g or less. Furthermore, the first and second backflow amount is preferably 85 g or less, more preferably 80 g or less.

<Backflow Test>

A measuring instrument including a cylinder for inputting liquid having an opening with an inner diameter of 3 cm is placed on a central portion of an absorbent article arranged on a horizontal stage. Next, 150 mL of the test liquid adjusted to 25 ± 1°C is input into the cylinder at once (supplied from the vertical direction). After 5 minutes from the start of input of the test liquid, filter paper of 10 cm square having a mass (We (g), about 80 g) measured in advance is placed near the input position of the test liquid on the absorbent article, and a weight having a mass of 5 kg and a bottom surface of 10 cm × 10 cm is placed on the filter paper. After applying the load for five minutes, the mass of the filter paper (Wf (g)) is measured, and the increased mass is taken as the liquid backflow amount (g). Also 15 minutes after the start of the first input of the test liquid, the same operation as in the first input is performed, and the total of the first and second inputs is taken as the total liquid backflow amount. The smaller the liquid backflow amount is, the more preferable the absorbent article is.

$$\text{Liquid backflow amount (g)} = \text{Wf} - \text{We}$$

The water-absorbent resin of the present invention is constituted of a product obtained by crosslinking a polymer of water-soluble ethylenically unsaturated monomers, that is, a crosslinked polymer having a structural unit derived from water-soluble ethylenically unsaturated monomers.

The water-absorbent resin used in the present invention may have various shapes. Examples of the shape of the water-absorbent resin include a granular shape, a substantially spherical shape, an indefinite crushed shape, a plate shape, a fibrous shape, a flake shape, and a shape in which these pieces of the resin are aggregated. The water-absorbent resin preferably has a granular shape, a substantially spherical shape, an indefinite crushed shape, a fibrous shape, a shape in which these pieces of the resin are aggregated, or the like.

The water-absorbent resin may be in a form in which fine particles (primary particles) are aggregated (secondary particles) in addition to a form in which particles of the water-absorbent resin are single particles. Examples of the shape of the primary particles include a substantially spherical shape, an indefinite crushed shape, and a plate shape. Examples of the primary particles produced by reversed phase suspension polymerization include substantially spherical single particles having a smooth surface shape such as a perfect spherical shape and an ellipsoidal shape. The primary particles having such a shape and having a smooth surface shape thus have high flowability as a powder, and aggregated particles are easily densely packed and thus are less likely to be broken even when subjected to impact, so that a water-absorbent resin having high particle strength is provided.

The median particle diameter of the water-absorbent resin is preferably 200 μm or more, 250 μm or more, 280 μm or more, 300 μm or more, or 320 μm or more from the viewpoint of more suitably exerting the effect of the present invention. From the same viewpoint, the median particle diameter is preferably 700 μmor less, 600 μm or less, 550 μm or less, 500 μm or less, 450 μm or less, or 400 μm or less. That is, the median particle diameter is preferably 200 the 700 μm, preferably 200 to 600 μm, more preferably 250 to 500 μm, still more preferably 300 to 450 μm, still more preferably 320 to 400 μm.

The median particle diameter of the water-absorbent resin can be measured using a JIS standard sieve. Specifically, the median particle diameter is a value measured by the method described in the EXAMPLES section.

As a polymerization method of the water-soluble ethylenically unsaturated monomers, an aqueous solution polymerization method, an emulsion polymerization method, a reversed phase suspension polymerization method, and the like, which are representative polymerization methods, are used. In the aqueous solution polymerization method, polymerization is performed by heating a water-soluble ethylenically unsaturated aqueous monomer solution while stirring the aqueous solution as necessary. In the reversed phase suspension polymerization method, polymerization is performed by heating water-soluble ethylenically unsaturated monomers in a hydrocarbon dispersion medium under stirring. The reversed phase suspension polymerization method is preferably used from the viewpoint of enabling precise control of the polymerization reaction and control over a wide particle diameter range.

A specific embodiment of a method for producing a water-absorbent resin will be described below using a reversed phase suspension polymerization method as an example.

Specific examples of the method for producing a water-absorbent resin include a method for producing a water-absorbent resin by performing reversed phase suspension polymerization of water-soluble ethylenically unsaturated monomers in a hydrocarbon dispersion medium, the method including a step of performing polymerization in the presence of a radical polymerization initiator and a step of post-crosslinking a hydrogel-like product obtained by the polymerization in the presence of a post-crosslinking agent. In the method for producing a water-absorbent resin of the present invention, an internal crosslinking agent may be added to the water-soluble ethylenically unsaturated monomers as necessary to form a hydrogel-like product having an internal crosslinked structure.

In order to set the repeated water absorption amount in an inclined state to 35 g or more in the water-absorbent resin of the present invention, for example, in the step of post-crosslinking in the presence of a post-crosslinking agent, it is preferable to adjust the amount of water in the hydrogel-like product at the time of post-crosslinking and the amount of the post-crosslinking agent to be added as described later. When the amount of water in the hydrogel-like product at the time of post-crosslinking is increased to a higher level than usual and the amount of the post-crosslinking agent added is decreased to a lower level than usual, the crosslink density can be decreased while increasing the crosslinking thickness of the surface portion of the hydrogel-like product, and the obtained water-absorbent resin has a high repeated water absorption amount in an inclined state of 35 g or more even with a similar absorption capacity, so that both suppression of backflow and suppression of leakage of the liquid absorbed by the absorbent body are suitably achieved.

<Polymerization Step>

[Water-Soluble Ethylenically Unsaturated Monomer]

Examples of the water-soluble ethylenically unsaturated monomers include (meth)acrylic acid (in the present specification, "acrylic" and "methacrylic" are collectively referred to as "(meth)acrylic". The same applies hereinafter) and salts thereof; 2-(meth)acrylamido-2-methylpropane sulfonic acid and salts thereof; nonionic monomers such as (meth)acrylamide, N,N-dimethyl(meth)acrylamide, 2-hydroxyethyl (meth)acrylate, N-methylol(meth)acrylamide, and polyethylene glycol mono(meth)acrylate; and amino group-containing unsaturated monomers such as N,N-diethylaminoethyl (meth)acrylate, N,N-diethylaminopropyl (meth)acrylate, and diethylaminopropyl(meth)acrylamide and quaternized products thereof. Among these water-soluble ethylenically unsaturated monomers, (meth)acrylic acid or a salt thereof, (meth)acrylamide, and N,N-dimethylacrylamide are preferable, and (meth)acrylic acid and a salt thereof are more preferable from the viewpoint of industrial availability and the like. These water-soluble ethylenically unsaturated monomers may be used singly or in combination of two or more kinds thereof.

Among them, acrylic acid and salts thereof are widely used as raw materials of water-absorbent resins, and these acrylic acid and/or a salt thereof may be copolymerized with another one of the above water-soluble ethylenically unsaturated monomers. In this case, acrylic acid and/or a salt thereof is preferably used as main water-soluble ethylenically unsaturated monomers in an amount of 70 to 100 mol% with respect to the total amount of water-soluble ethylenically unsaturated monomers.

The water-soluble ethylenically unsaturated monomers are preferably dispersed in a hydrocarbon dispersion medium in a state of an aqueous solution and subjected to reversed phase suspension polymerization. When the water-soluble ethylenically unsaturated monomers are in an aqueous solution, the dispersion efficiency in the hydrocarbon dispersion medium can be increased. The concentration of the water-soluble ethylenically unsaturated monomers in this aqueous solution is preferably in the range of 20 mass% to a saturated concentration. The concentration of the water-soluble ethylenically unsaturated monomers is more preferably 55 mass% or less, still more preferably 50 mass% or less, still more preferably 45 mass% or less. On the other hand, the concentration of the water-soluble ethylenically unsaturated monomers is more preferably 25 mass% or more, still more preferably 28 mass% or more, still more preferably 30 mass% or more.

When the water-soluble ethylenically unsaturated monomers have acid groups such as (meth)acrylic acid and 2-(meth)acrylamido-2-methylpropane sulfonic acid, a compound in which the acid group has been neutralized in advance with an alkaline neutralizing agent as necessary may be used. Examples of such an alkaline neutralizing agent include alkali metal salts such as sodium hydroxide, sodium carbonate, sodium hydrogen carbonate, potassium hydroxide, and potassium carbonate; and ammonia. In addition, these alkaline neutralizing agents may be used in the form of an aqueous solution in order to simplify the neutralization operation. The above alkaline neutralizing agents may be used singly or in combination of two or more kinds thereof.

The neutralization degree of the water-soluble ethylenically unsaturated monomers with the alkaline neutralizing agent is preferably 10 to 100 mol%, more preferably 30 to 90 mol%, still more preferably 40 to 85 mol%, still more preferably 50 to 80 mol%, as the neutralization degree with respect to all the acid groups of the water-soluble ethylenically unsaturated monomers.

[Radical Polymerization Initiator]

Examples of the radical polymerization initiator added in the polymerization step include persulfates such as potassium persulfate, ammonium persulfate, and sodium persulfate, peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide, and azo compounds such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, and 2,2'-azobis{2-[1-(2-hydroxyethyl)-2-imidazolin-2-yl]propane dihydrochloride, 2,2'-azobis{2-methyl-N-[1,1-bis(hydroxymethyl)-2-hydroxyethyl]propionamide], 2,2'-azobis[2-methyl-N-(2-hydroxyethyl)-propionamide], and 4,4'-azobis(4-cyanovaleric acid). Among these radical polymerization initiators, potassium persulfate, ammonium persulfate, sodium persulfate, and 2,2'-azobis(2-amidinopropane) dihydrochloride are preferable from the viewpoint of easy availability and easy handling. These radical polymerization initiators may be used singly or in combination of two or more. The radical polymerization initiator can also be used as a redox polymerization initiator in combination with a reducing agent such as sodium sulfite, sodium hydrogen sulfite, ferrous sulfate, and L-ascorbic acid.

The amount of the radical polymerization initiator used is, for example, 0.00005 to 0.01 mol with respect to 1 mol of the water-soluble ethylenically unsaturated monomers. By satisfying such a use amount, occurrence of a rapid polymerization reaction can be avoided, and the polymerization reaction can be completed in an appropriate time.

[Internal Crosslinking Agent]

Examples of the internal crosslinking agent include those capable of crosslinking a polymer of water-soluble ethylenically unsaturated monomers to be used, such as unsaturated polyesters obtained by reacting polyols such as diols and triols such as (poly)ethylene glycol ["(poly)" means with or without a "poly" prefix. The same applies hereinafter], (poly)propylene glycol, 1,4-butanediol, trimethylolpropane, and (poly)glycerol with an unsaturated acid such as (meth)acrylic acid, maleic acid, and fumaric acid; bisacrylamides such as N,N-methylenebisacrylamide; di(meth)acrylic acid esters or tri(meth)acrylic acid esters obtained by reacting a polyepoxide with (meth)acrylic acid; di(meth)acrylic acid carbamyl esters obtained by reacting polyisocyanate such as tolylene diisocyanate and hexamethylene diisocyanate with hydroxyethyl (meth)acrylate; compounds having two or more polymerizable unsaturated groups, such as allylated starch, allylated cellulose, diallyl phthalate, N,N',N''-triallyl isocyanurate, and divinylbenzene; polyglycidyl compounds such as diglycidyl compounds such as (poly)ethylene glycol diglycidyl ether, (poly)propylene glycol diglycidyl ether, and (poly)glycerol diglycidyl ether and triglycidyl compounds; epihalohydrin compounds such as epichlorohydrin, epibromohydrin, and α-methylepichlorohydrin; compounds having two or more reactive functional groups such as isocyanate compounds such as 2,4-tolylene diisocyanate and hexamethylene diisocyanate; and oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol. Among these internal crosslinking agents, an unsaturated polyester or a polyglycidyl compound is preferably used, a diglycidyl ether compound is more preferably used, and (poly)ethylene glycol diglycidyl ether, (poly)propylene glycol diglycidyl ether, and (poly)glycerol diglycidyl ether are preferably used. These internal crosslinking agents may be used singly or in combination of two or more.

The amount of the internal crosslinking agent used is preferably 0.000001 to 0.02 mol, more preferably 0.00001 to 0.01 mol, still more preferably 0.00001 to 0.005 mol, still more preferably 0.00005 to 0.002 mol, with respect to 1 mol of the water-soluble ethylenically unsaturated monomers.

[Hydrocarbon Dispersion Medium]

Examples of the hydrocarbon dispersion medium include aliphatic hydrocarbons having six to eight carbon atoms such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. Among these hydrocarbon dispersion media, n-hexane, n-heptane, and cyclohexane are particularly preferably used because they are industrially easily available, have stable quality, and are inexpensive. These hydrocarbon dispersion media may be used singly or in combination of two or more. As an example of a mixture of the hydrocarbon dispersion media, a suitable result can be obtained by using a commercially available product such as Exxsol Heptane (manufactured by Exxon Mobil Corporation: containing 75 to 85 mass% of heptane and an isomer hydrocarbon thereof).

The amount of the hydrocarbon dispersion medium used is preferably 100 to 1,500 parts by mass, more preferably 200 to 1,400 parts by mass, with respect to 100 parts by mass of the first-step water-soluble ethylenically unsaturated monomers from the viewpoint of uniformly dispersing the water-soluble ethylenically unsaturated monomers and facilitating the control of the polymerization temperature. As will be described later, the reversed phase suspension polymerization is performed in one step (single step) or multiple steps of two or more steps, and the above-described first-step polymerization means the first-step polymerization reaction in the single-step polymerization or the multi-step polymerization (the same applies hereinafter).

[Dispersion Stabilizer]

(Surfactant)

In the reversed phase suspension polymerization, a dispersion stabilizer can also be used in order to improve the dispersion stability of the water-soluble ethylenically unsaturated monomers in the hydrocarbon dispersion medium. As the dispersion stabilizer, a surfactant can be used.

Examples of the surfactant used include a sucrose fatty acid ester, a polyglycerol fatty acid ester, a sorbitan fatty acid ester, a polyoxyethylene sorbitan fatty acid ester, a polyoxyethylene glycerol fatty acid ester, a sorbitol fatty acid ester, a polyoxyethylene sorbitol fatty acid ester, a polyoxyethylene alkyl ether, a polyoxyethylene alkyl phenyl ether, a polyoxyethylene castor oil, a polyoxyethylene hydrogenated castor oil, an alkylallyl formaldehyde condensed polyoxyethylene ether, a polyoxyethylene polyoxypropylene block copolymer, a polyoxyethylene polyoxypropyl alkyl ether, a polyethylene glycol fatty acid ester, an alkyl glucoside, an N-alkyl gluconamide, a polyoxyethylene fatty acid amide, a polyoxyethylene alkylamine, a phosphoric acid ester of a polyoxyethylene alkyl ether, and a phosphoric acid ester of a polyoxyethylene alkyl allyl ether. Among these surfactants, it is particularly preferable to use a sorbitan fatty acid ester, a polyglycerol fatty acid ester, or a sucrose fatty acid ester from the viewpoint of dispersion stability of the monomers. These surfactants may be used singly or in combination of two or more.

The amount of the surfactant used is preferably 0.1 to 30 parts by mass, more preferably 0.3 to 20 parts by mass, with respect to 100 parts by mass of the first-step water-soluble ethylenically unsaturated monomers.

(Polymeric Dispersant)

As the dispersion stabilizer used in the reversed phase suspension polymerization, a polymeric dispersant may be used together with the surfactant described above.

Examples of the polymeric dispersant include maleic anhydride-modified polyethylene, maleic anhydride-modified polypropylene, maleic anhydride-modified ethylene-propylene copolymers, maleic anhydride-modified EPDM (ethylene-propylene-diene terpolymers), maleic anhydride-modified polybutadiene, maleic anhydride-ethylene copolymers, maleic anhydride-propylene copolymers, maleic anhydride-ethylene-propylene copolymers, maleic anhydride-butadiene copolymers, polyethylene, polypropylene, ethylene-propylene copolymers, oxidized polyethylene, oxidized polypropylene, oxidized ethylene-propylene copolymers, ethylene-acrylic acid copolymers, ethyl cellulose, and ethyl hydroxyethyl cellulose. Among these polymeric dispersants, maleic anhydride-modified polyethylene, maleic anhydride-modified polypropylene, maleic anhydride-modified ethylene-propylene copolymers, maleic anhydride-ethylene copolymers, maleic anhydride-propylene copolymers, maleic anhydride-ethylene-propylene copolymers, polyethylene, polypropylene, ethylene-propylene copolymers, oxidized polyethylene, oxidized polypropylene, and oxidized ethylene-propylene copolymers are particularly preferably used from the viewpoint of dispersion stability of the monomers. These polymeric dispersants may be used singly or in combination of two or more.

The amount of the polymeric dispersant used is preferably 0.1 to 30 parts by mass, more preferably 0.3 to 20 parts by mass, with respect to 100 parts by mass of the first-step water-soluble ethylenically unsaturated monomers.

[Other Components]

In the method for producing a water-absorbent resin, if desired, other components may be added to an aqueous solution containing the water-soluble ethylenically unsaturated monomers to perform reversed phase suspension polymerization. As other components, various additives such as a thickener and a chain transfer agent can be added.

As an example, reversed phase suspension polymerization can be performed with a thickener added to an aqueous solution containing the water-soluble ethylenically unsaturated monomers. Adding a thickener as described above to adjust the viscosity of the aqueous solution enables control of the median particle diameter obtained by the reversed phase suspension polymerization.

As the thickener, for example, hydroxyethyl cellulose, hydroxypropyl cellulose, methyl cellulose, carboxymethyl cellulose, polyacrylic acid, (partially) neutralized polyacrylic acid, polyethylene glycol, polyacrylamide, polyethyleneimine, dextrin, sodium alginate, polyvinyl alcohol, polyvinylpyrrolidone, polyethylene oxide, or the like can be used. When the stirring rate during polymerization is constant, the primary particles and/or the secondary particles of the resulting particles tend to be larger as the viscosity of the aqueous solution of the water-soluble ethylenically unsaturated monomers is higher.

[Reversed Phase Suspension Polymerization]

In performing the reversed phase suspension polymerization, for example, a aqueous monomer solution containing the water-soluble ethylenically unsaturated monomers is dispersed in a hydrocarbon dispersion medium in the presence of a dispersion stabilizer. At this time, as long as it is before the polymerization reaction is started, the addition timing of the dispersion stabilizer (surfactant or polymeric dispersant) may be either before or after the addition of the aqueous monomer solution.

Among them, from the viewpoint of easily reducing the amount of the hydrocarbon dispersion medium remaining in the resulting water-absorbent resin, it is preferable to perform polymerization after dispersing the surfactant in the hydrocarbon dispersion medium in which the polymeric dispersant has been dispersed and in which the aqueous monomer solution has then been dispersed.

Such reversed phase suspension polymerization can be performed in one step or multiple steps of two or more steps. In addition, from the viewpoint of enhancing productivity, it is preferable to employ two or three steps.

When the reversed phase suspension polymerization is performed in two or more steps, the first-step reversed phase suspension polymerization is performed, then the water-soluble ethylenically unsaturated monomers are added to and mixed with the reaction mixture obtained in the first-step polymerization reaction, and the second-step or subsequent reversed phase suspension polymerization may be performed in the same manner as the first-step polymerization. In the reversed phase suspension polymerization in each of the second and subsequent steps, in addition to the water-soluble ethylenically unsaturated monomers, a radical polymerization initiator is preferably added within the range of the molar ratio of each component to the water-soluble ethylenically unsaturated monomers described above based on the amount of the water-soluble ethylenically unsaturated monomers added in the reversed phase suspension polymerization in each of the second and subsequent steps to perform reversed phase suspension polymerization. In the second and subsequent polymerization, an internal crosslinking agent may be added to the water-soluble ethylenically unsaturated monomers as necessary.

The reaction temperature of the polymerization reaction is preferably 20 to 110°C, more preferably 40 to 90°C, from the viewpoint of enhancing the economic efficiency by rapidly progressing the polymerization and shortening the polymerization time and easily removing the heat of polymerization to smoothly perform the reaction.

<Post-Crosslinking Step>

Next, the water-absorbent resin of the present invention is obtained by adding a post-crosslinking agent to a hydrogel-like product having an internally crosslinked structure obtained by polymerizing the water-soluble ethylenically unsaturated monomers, and crosslinking the hydrogel-like product (post-crosslinking reaction). This post-crosslinking reaction is preferably performed in the presence of a post-crosslinking agent after polymerization of the water-soluble ethylenically unsaturated monomers. As described above, by subjecting the hydrogel-like product having an internally crosslinked structure to a post-crosslinking reaction after polymerization, it is possible to obtain a water-absorbent resin in which the crosslinking density in the vicinity of the surface of the water-absorbent resin is appropriately increased and various performances such as the repeated water absorption amount in an inclined state are improved.

As described above, in order to set the repeated water absorption amount in an inclined state to 35 g or more in the water-absorbent resin of the present invention, for example, in the step of post-crosslinking in the presence of a post-crosslinking agent, it is preferable to adjust the amount of water in the hydrogel-like product at the time of post-crosslinking and the amount of the post-crosslinking agent to be added as described later. When the amount of water in the hydrogel-like product at the time of post-crosslinking is increased to a higher level than usual and the amount of the post-crosslinking agent added is decreased to a lower level than usual, the crosslink density can be decreased while increasing the crosslinking thickness of the surface portion of the hydrogel-like product, and the obtained water-absorbent resin has a high repeated water absorption amount in an inclined state of 35 g or more even with a similar absorption capacity, so that both suppression of backflow and suppression of leakage of the liquid absorbed by the absorbent body are suitably achieved.

In the water-absorbent resin of the present invention, from the viewpoint of suitably setting the repeated water absorption amount in an inclined state to 35 g or more, as for the amount of water in the hydrogel-like product at the time of post-crosslinking, addition is preferably performed in the presence of water in the range of 5 to 140 parts by mass, more preferably in the presence of water in the range of 15 to 100 parts by mass, still more preferably in the presence of water in the range of 20 to 50 parts by mass, still more preferably in the presence of water in the range of 22 to 31 parts by mass, with respect to 100 parts by mass of the water-soluble ethylenically unsaturated monomers. From the same viewpoint, the added amount of the post-crosslinking agent on the surface of the hydrogel-like product is preferably 0.00001 to 0.01 mol, more preferably 0.00002 to 0.001 mol, still more preferably 0.00005 to 0.0005 mol, on the basis of a total of 1 mol of the water-soluble ethylenically unsaturated monomers used for polymerization.

Examples of the post-crosslinking agent include compounds having two or more reactive functional groups. Examples include polyols such as ethylene glycol, propylene glycol, 1,4-butanediol, trimethylolpropane, glycerol, polyoxyethylene glycol, polyoxypropylene glycol, and polyglycerol; polyglycidyl compounds such as (poly)ethylene glycol diglycidyl ether, (poly)glycerol diglycidyl ether, (poly)glycerol triglycidyl ether, trimethylolpropane triglycidyl ether, (poly)propylene glycol polyglycidyl ether, and (poly)glycerol polyglycidyl ether; haloepoxy compounds such as epichloro-hydrin, epibromohydrin, and $\alpha$-methylepichlorohydrin; isocyanate compounds such as 2,4-tolylene diisocyanate and hexamethylene diisocyanate; oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol; oxazoline compounds such as 1,2-ethylenebisoxazoline; carbonate compounds such as ethylene carbonate; and hydroxyalkylamide compounds such as bis[N,N-di($\beta$-hydroxyethyl)]adipamide. Among these post-crosslinking agents, polyglycidyl compounds such as (poly)ethylene glycol diglycidyl ether, (poly)glycerol diglycidyl ether, (poly)glycerol triglycidyl ether, trimethylolpropane triglycidyl ether, (poly)propylene glycol polyglycidyl ether, and (poly)glycerol polyglycidyl ether are preferable. These post-crosslinking agents may be used singly or in combination of two or more.

The amount of the post-crosslinking agent used is preferably 0.00001 to 0.01 mol, more preferably 0.00002 to 0.001 mol, still more preferably 0.00005 to 0.0005 mol, with respect to a total of 1 mol of the water-soluble ethylenically unsaturated monomers used for the polymerization.

As a method for adding the post-crosslinking agent, the post-crosslinking agent may be added as it is or in the form of an aqueous solution or may be added in the form of a solution containing a hydrophilic organic solvent as a solvent as necessary. Examples of the hydrophilic organic solvent include lower alcohols such as methyl alcohol, ethyl alcohol, n-propyl alcohol, and isopropyl alcohol; ketones such as acetone and methyl ethyl ketone; ethers such as diethyl ether, dioxane, and tetrahydrofuran; amides such as N,N-dimethylformamide; and sulfoxides such as dimethyl sulfoxide. These hydrophilic organic solvents may be used singly or in combination of two or more or may be used in the form of a mixed solvent with water.

The addition timing of the post-crosslinking agent is after almost all the polymerization reaction of the water-soluble ethylenically unsaturated monomers is completed, and the post-crosslinking agent is preferably added in the presence of water in the range of 5 to 140 parts by mass, more preferably in the presence of water in the range of 15 to 100 parts by mass, still more preferably in the presence of water in the range of 20 to 50 parts by mass, still more preferably in the presence of water in the range of 22 to 31 parts by mass, with respect to 100 parts by mass of the water-soluble ethylenically unsaturated monomers. The amount of water means the total amount of water contained in the reaction system and water used as necessary when the post-crosslinking agent is added. When the post-crosslinking agent is added in a state in which the amount of water is greater than 140 parts by mass, the water retention tends to decrease. In addition, when the post-crosslinking agent is added in a state in which the amount of water is less than 5 parts by mass, the reaction of the post-crosslinking agent tends to be insufficient.

The reaction temperature in the post-crosslinking reaction is preferably 50 to 250°C, more preferably 60 to 180°C, still more preferably 60 to 140°C, still more preferably 70 to 120°C. The reaction time of the post-crosslinking reaction is preferably 1 to 300 minutes, more preferably 5 to 200 minutes.

<Drying Step>

The method may include a drying step of removing water, a hydrocarbon dispersion medium, and the like by distillation by externally applying energy such as heat after performing the reversed phase suspension polymerization described above. When dehydration is performed from the hydrogel after the reversed phase suspension polymerization, the system in which the hydrogel is dispersed in the hydrocarbon dispersion medium is heated, so that water and the hydrocarbon dispersion medium are temporarily distilled off to the outside of the system by azeotropic distillation. At this time, when only the distilled hydrocarbon dispersion medium is returned into the system, continuous azeotropic distillation becomes possible. In that case, since the temperature in the system during drying is maintained at an azeotropic temperature with the hydrocarbon dispersion medium or lower, that case is preferable from the viewpoint that the resin is hardly deteriorated. Subsequently, water and the hydrocarbon dispersion medium are distilled off to obtain the water-absorbent resin. Various performances of the water-absorbent resin to be obtained can be controlled by controlling the treatment conditions in the drying step after the polymerization to adjust the amount of water to be removed.

In the drying step, the drying treatment by distillation may be performed under normal pressure or under reduced pressure. From the viewpoint of enhancing the drying efficiency, the drying may be performed under a flow of nitrogen or the like. The drying temperature when the drying treatment is performed under normal pressure is preferably 70 to 250°C, more preferably 80 to 180°C, still more preferably 80 to 140°C, still more preferably 90 to 130°C. When the drying treatment is performed under reduced pressure, the drying temperature is preferably 40 to 160°C, more preferably 50 to 110°C.

When the post-crosslinking step with the post-crosslinking agent is performed after the polymerization of the monomers is performed by reversed phase suspension polymerization, the drying step by distillation described above is performed after the completion of the post-crosslinking step. Alternatively, the post-crosslinking step and the drying step may be performed simultaneously.

The water-absorbent resin of the present invention may contain an additive according to the purpose. Examples of such an additive include inorganic powders, surfactants, oxidizing agents, reducing agents, metal chelating agents, radical chain inhibitors, antioxidants, and antibacterial agents. For example, by adding 0.05 to 5 parts by mass of amorphous silica as an inorganic powder to 100 parts by mass of the water-absorbent resin, the flowability of the water-absorbent resin can be further improved.

2. Absorbent Body and Absorbent Article

The water-absorbent resin of the present invention constitutes an absorbent body used for sanitary supplies such as sanitary products and disposable diapers and is suitably used for an absorbent article including the absorbent body.

The absorbent body of the present invention contains the water-absorbent resin of the present invention. The absorbent body may further contain hydrophilic fibers. Examples of the configuration of the absorbent body include a sheet-like structure in which the water-absorbent resin is fixed on a non-woven fabric or between a plurality of non-woven fabrics, a mixed dispersion obtained by mixing the water-absorbent resin and hydrophilic fibers so as to have a uniform composition, a sandwich structure in which the water-absorbent resin is sandwiched between layered hydrophilic fibers, and a structure in which the water-absorbent resin and hydrophilic fibers are wrapped with tissue. The absorbent body may contain other components, such as an adhesive binder such as a heat-sealable synthetic fiber, a hot melt adhesive, and an adhesive emulsion for enhancing the shape retention of the absorber.

The weight of the water-absorbent resin per unit area in the absorbent body of the present invention is 50 g/m$^2$ or more and 400 g/m$^2$ or less. From the viewpoint of more suitably exerting the effect of the present invention, the weight per unit area is preferably 100 g/m$^2$ or more, more preferably 120 g/m$^2$ or more, still more preferably 140 g/m$^2$ or more, and is preferably 300 g/m$^2$ or less, more preferably 250 g/m$^2$ or less, still more preferably 200 g/m$^2$ or less.

The content of the water-absorbent resin in the absorbent body is preferably 5 to 100 mass%, more preferably 10 to 95 mass%, still more preferably 20 to 90 mass%, still more preferably 30 to 80 mass%.

Examples of the hydrophilic fibers include at least one selected from the group consisting of finely pulverized wood pulp, cotton, cotton linters, rayon, cellulose acetate, polyamides, polyesters, and polyolefins. Examples include cellulose fibers such as cotton-like pulp, mechanical pulp, chemical pulp, and semi-chemical pulp obtained from wood, artificial cellulose fibers such as rayon and acetate, and fibers made of synthetic resins such as hydrophilized polyamide, polyesters, and polyolefins. The average fiber length of the hydrophilic fibers is usually 0.1 to 10 mm or may be 0.5 to 5 mm.

The absorbent body using the water-absorbent resin of the present invention can be held between a liquid-permeable sheet (top sheet) through which liquid can pass and a liquid-impermeable sheet (back sheet) through which liquid cannot pass to form the absorbent article of the present invention. The liquid-permeable sheet is disposed on the side in contact with the body, and the liquid-impermeable sheet is disposed on the side opposite to the side in contact with the body.

Examples of the liquid-permeable sheet include non-woven fabrics of an air through type, a spunbond type, a chemical bond type, a needle punch type, or the like made of fibers of polyethylene, polypropylene, a polyester, or the like and porous synthetic resin sheets. Examples of the liquid-impermeable sheet include synthetic resin films made of resins such as polyethylene, polypropylene, and polyvinyl chloride. The liquid-permeable sheet is preferably at least one selected from the group consisting of a thermally bonded non-woven fabric, an air through non-woven fabric, a spunbonded non-woven fabric, and a spunbonded/meltblown/spunbonded (SMS) non-woven fabric.

The weight per unit area of the liquid-permeable sheet is preferably 5 g/m$^2$ or more and 100 g/m$^2$ or less, more preferably 10 g/m$^2$ or more and 60 g/m$^2$ or less. The surface of the liquid-permeable sheet may be embossed or perforated in order to improve the liquid diffusibility. The embossing and the perforating can be performed by a known method.

Examples of the liquid-impermeable sheet include a sheet made of a synthetic resin such as polyethylene, polypropylene, and polyvinyl chloride, a sheet made of a non-woven fabric such as an SMS nonwoven fabric obtained by sandwiching a water-resistant meltblown non-woven fabric with high-strength spunbonded non-woven fabrics, and a sheet made of a composite material of these synthetic resins and a non-woven fabric (such as a spunbonded non-woven fabric and a spunlace non-woven fabric). As the liquid-impermeable sheet, a sheet made of a synthetic resin mainly containing a low density polyethylene (LDPE) resin can also be used. The liquid-impermeable sheet may be, for example, a sheet made of a synthetic resin having a weight per unit area of 10 to 50 g/m2.

The absorbent article preferably includes a laminate including the absorbent body containing the water-absorbent resin and core wrap disposed on the upper and lower sides of the absorbent body to sandwich the absorbent body therebetween, a liquid-permeable sheet disposed on an upper surface of the laminate, and a liquid-impermeable sheet disposed on a surface of the laminate on a side opposite to the liquid-permeable sheet side.

EXAMPLES

Hereinafter, the present invention will be described in detail with reference to examples and comparative examples. However, the present invention is not limited to the examples.

The water-absorbent resins obtained in the examples and comparative examples were evaluated through the following various tests. Unless otherwise specified, the measurement was performed under an environment of a temperature of 25 $\pm$ 2°C and a humidity of 50 $\pm$ 10%. Hereinafter, each evaluation test method will be described.

<Measurement of Physiological Saline Retention>

A cotton bag (No. 60 count cotton broadcloth, 100 mm in width $\times$ 200 mm in length) in which 2.0 g of the water-absorbent resin had been weighed out was placed in a 500-mL beaker. Into the cotton bag containing the water-absorbent resin, 500 g of a 0.9-mass% aqueous sodium chloride solution (physiological saline) was poured at once so as not to form lumps, the upper portion of the cotton bag was tied with a rubber band, and the cotton bag was left to stand for 30 minutes to allow the water-absorbent resin to swell. The cotton bag after 30 minutes was dehydrated for 1 minute using a dehydrator (manufactured by KOKUSAN Co. Ltd., product No. H-122) set so that the centrifugal force was 167 G, and the mass Wa (g) of the cotton bag containing the swollen gel after dehydration was measured. The same operation was performed without adding the water-absorbent resin, the empty mass Wb (g) of the cotton bag in a wet state was measured, and the physiological saline retention was calculated from the following equation.

$$\text{Physiological saline retention (g/g)} = [\text{Wa - Wb}]/2.0$$

<Measurement of Five-Minute Non-Pressurized DW (Demand Wettability)>

The non-pressurized DW of particles of the water-absorbent resin was measured using a measuring device shown in Fig. 1. The measurement was performed five times for one kind of water-absorbent resin, and the average value of the measured values of three points excluding the minimum value and the maximum value was determined. The measuring device includes the burette unit 1, the conduit 5, the measurement stage 13, the nylon mesh sheet 15, the stand 11, and the clamp 3. The burette unit 1 includes the graduated burette tube 21, the rubber stopper 23 for tightly sealing the upper opening of the burette tube 21, the cock 22 connected to the lower tip of the burette tube 21, and the air introduction tube 25 and the cock 24 connected to the lower portion of the burette tube 21. The burette unit 1 is fixed with the clamp 3. The flat plate-shaped measurement stage 13 has the through hole 13a having a diameter of 2 mm formed in a central portion thereof and is supported by the height-adjustable stand 11. The through hole 13a of the measurement stage 13 and the cock 22 of the burette unit 1 are connected by the conduit 5. The inner diameter of the conduit 5 is 6 mm.

First, the cock 22 and the cock 24 of the burette unit 1 were closed, and the 0.9-mass% saline 50 adjusted to 25°C was put into the burette tube 21 from the opening of the upper portion of the burette tube 21. The concentration of the saline of 0.9 mass% is a concentration based on the mass of the saline. After the opening of the burette tube 21 was tightly sealed with the rubber stopper 23, the cock 22 and the cock 24 were opened. The inside of the conduit 5 was filled with the 0.9-mass% saline 50 so that air bubbles did not enter. The height of the measurement stage 13 was adjusted such that the height of the surface of the 0.9-mass% saline reaching the inside of the through hole 13a became equal to the height of the upper surface of the measurement stage 13. After the adjustment, the height of the surface of the 0.9-mass% saline 50 in the burette tube 21 was read using the graduations on the burette tube 21, and the position was set as a 0 point (reading at 0 seconds).

The nylon mesh sheet 15 (100 mm × 100 mm, 250 mesh, thickness of about 50 μm) was laid in the vicinity of the through hole 13a on the measurement stage 13, and a cylinder having an inner diameter of 30 mm and a height of 20 mm was placed at the center thereof. In the cylinder, 1.00 g of a water-absorbent resin 10a was uniformly spread. Thereafter, the cylinder was carefully removed to obtain a sample in which the water-absorbent resin 10a was circularly dispersed on the central portion of the nylon mesh sheet 15. Next, the nylon mesh sheet 15 on which the water-absorbent resin 10a was placed was moved quickly to such an extent that the water-absorbent resin 10a was not scattered so that the center of the nylon mesh sheet 15 was located at the position of the through hole 13a, and measurement was started. A point of time when air bubbles were first introduced from the air introduction tube 25 into the burette tube 21 was defined as the start of water absorption (0 seconds).

A decrease amount (that is, the amount of the 0.9-mass% saline absorbed by the water-absorbent resin 10a) of the 0.9-mass% saline 50 in the burette tube 21 was sequentially read, and the decrement Wc (g) of the 0.9-mass% saline 50 after 5 minutes from the start of water absorption by the water-absorbent resin 10a was read. From the decrement Wc, the five-minute non-pressurized DW was obtained by the following equation. The non-pressurized DW is a water absorption amount per 1.00 g of the water-absorbent resin 10a.

$$\text{Non-pressurized DW value (mL/g)} = \text{Wc}/1.00$$

<Method for Measuring Physiological Saline Absorption Rate>

The physiological saline absorption rate of the water-absorbent resin was measured by the following procedure based on the vortex method. First, 50 ± 0.1 g of physiological saline adjusted to a temperature of 25 ± 0.2°C in a constant temperature water bath was weighed out in a beaker having an internal volume of 100 mL. Next, the physiological saline was stirred at a rotation speed of 600 rpm using a magnetic stir bar (8 mmφ × 30 mm, without ring) to generate a vortex. To the physiological saline, 2.0 ± 0.002 g of the water-absorbent resin was added at once. The time [sec] from the addition of the water-absorbent resin to the time point at which the vortex on the liquid surface converged was measured, and the time was taken as the water absorption speed of the water-absorbent resin.

<Method for Measuring Median Particle Diameter>

Using an automated sonic sieving particle size analyzer (Robot Sifter RPS-205, manufactured by Seishin Enterprise Co., Ltd.), sieves with aperture sizes of 850 μm, 710 μm, 600 μm, 500 μm, 425 μm, 300 μm, 250 μm, and 150 μm conforming to JIS standards, and a pan, 10 g of the particulate water-absorbent resin was sieved. The mass of the particles remaining on each sieve was calculated as a mass percentage with respect to the total amount. The mass percentages of the particles remaining on the respective sieves were integrated in the descending order of particle diameters, and the relationship between the aperture size of the sieve and the integrated value of the mass percentage of the particles remaining on the sieve was plotted on log-probability paper. The plots on the probability paper were connected by a straight line to determine the particle diameter corresponding to an integrated mass percentage of 50 mass%, and the determined value was defined as the median particle diameter.

<Method for Measuring Repeated Water Absorption Amount in Inclined State>

The repeated water absorption amount in an inclined state of the water-absorbent resin was evaluated through the following procedures I), II), III), and IV).

I) In a petri dish made of plastic and having a circular shape, an inner diameter of 8.5 cm, and a height of 1.7 cm, 1.0 g of the water-absorbent resin was uniformly spread. Subsequently, 30 g of physiological saline was supplied at once on the water-absorbent resin. Immediately thereafter, the petri dish was shaken 10 times from side to side on a horizontal plane and then allowed to stand for 3 minutes to form a swollen gel layer in which the physiological saline was absorbed by the water-absorbent resin.

II) A device was prepared including an acrylic plate having a rectangular flat main surface with a length of 30 cm and a width of 55 cm fixed in such a manner that the width direction thereof was parallel to the horizontal plane and the main surface was at an angle of 30° to the horizontal plane. On an upper surface of the swollen gel layer, an air through non-woven fabric (Rengo Nonwoven Products Co., Ltd., material composition: 50% PP and 50% PE) having a weight per unit area of 21 g/m and cut into the same size as the inside of the petri dish was placed, and the petri dish was fixed to the acrylic plate with tape to avoid slipping off.

III) Using a macropipette (macropipette manufactured by Sibata Scientific Technology Ltd., capacity: 10 ml), 5 g of physiological saline at a liquid temperature of 25°C from a vertical height of about 1 cm from the central part of the swollen gel layer in the petri dish was poured for 5 seconds.

IV) The operation of III) was repeated eight times at an interval of one minute from the start of pouring of the physiological saline, and an amount Wd of liquid leaked from the petri dish was measured. From the amount Wd, the repeated water absorption amount in an inclined state was determined from the following equation.

$$\text{Repeated water absorption amount in inclined state (g/g)} = (40 - Wd)/1.00$$

<Production of Absorbent Body and Absorbent Article>

A sheet-shaped absorbent body having a size of 12 cm × 32 cm was produced by uniformly mixing 5.76 g of the water-absorbent resin and 5.76 g of pulverized pulp by air papermaking using an air flow type mixer (Padformer manufactured by O-tec Co., Ltd.). The absorbent body was disposed on tissue paper (core wrap) having a basis weight of 16 g/m$^2$, and tissue paper (core wrap) was laminated on the absorbent body in this order. A load of 196 kPa was applied to the laminate for 30 seconds. Further, a polyethylene liquid-impermeable sheet having a size of 12 cm × 32 cm was attached to a surface opposite to the breathable non-woven fabric to produce a test absorbent article. The weight per unit area of the polyethylene liquid-impermeable sheet used was 40 g/m$^2$, and the weight per unit area of the breathable non-woven fabric was 21 g/m$^2$. In the absorbent article, the basis weight of the water-absorbent resin was 150 g/m$^2$, and the basis weight of the pulverized pulp (hydrophilic fibers) was 150 g/m$^2$.

<Preparation of Test Liquid>

A test liquid was prepared by blending and dissolving an inorganic salt in ion-exchanged water so that the inorganic salt was present as described below and further blending a small amount of Brilliant Blue FCF.

Composition of test liquid

- Deionized water: 5919.6 g
- NaCl: 60.0 g
- CaCl$_2$·H$_2$O: 1.8 g
- MgCl$_2$·6H$_2$O: 3.6 g
- Brilliant Blue FCF for food (for coloring)
- 1% Triton X-100: 15.0 g

<Leakage Test (Gradient Absorption Test)>

Fig. 2 is a schematic diagram for illustrating a method for evaluating a leakage property of the absorbent article. The supporting plate 40 (an acrylic resin plate in this case, hereinafter also referred to as the inclined surface S$_1$) having a flat main surface and a length of 45 cm was fixed by the stand 41 in a state of being inclined at 45 ± 1° with respect to the horizontal plane S$_0$. The test absorbent article 100 (15.1 g) was stuck on the inclined surface S$_1$ of the fixed supporting plate 40 in such an orientation that the longitudinal direction thereof is along the longitudinal direction of the supporting plate 40. Next, the test liquid 51 adjusted to 25 ± 1°C was dropped from the dropping funnel 42 disposed vertically above the absorbent article toward a position 8 cm above the center of the absorbent body in the absorbent article 100. At a rate of 8 mL/sec, 80 mL per one time of the test liquid was added dropwise. The distance between the tip of the dropping funnel 42 and the absorbent article was 10 ± 1 mm. The test liquid was repeatedly input under the same conditions at intervals of 10 minutes from the start of the first input of the test liquid. The test liquid was input until leakage was observed, and the number of times of input was defined as the number of times of input until occurrence of leakage.

When the test liquid that had not been absorbed by the absorbent article 100 leaked from the lower portion of the supporting plate 40, the leaked test liquid was collected in the metal tray 44 disposed below the supporting plate 40. The mass (g) of the collected test liquid was measured with the balance 43, and the value was recorded as the leakage amount. The absorption amount until leakage occurred was calculated by subtracting the leakage amount from the total input amount of the test liquid. As this numerical value is larger, leakage of liquid is judged to be less likely to occur when the absorbent article is worn. The density of the test liquid was supposed to be 1.0 g/mL.

<Backflow Test>

A measuring instrument including a cylinder for inputting liquid having an opening with an inner diameter of 3 cm was placed on a central portion of the absorbent article arranged on a horizontal stage. Next, 150 mL of the test liquid adjusted to 25 ± 1°C was input into the cylinder at once (supplied from the vertical direction). After 5 minutes from the start of input of the test liquid, filter paper of 10 cm square having a mass (We (g), about 80 g) measured in advance was placed near the input position of the test liquid on the absorbent article, and a weight having a mass of 5 kg and a bottom surface of 10 cm × 10 cm was placed on the filter paper. After applying the load for five minutes, the mass of the filter paper (Wf (g)) was measured, and the increased mass was taken as the liquid backflow amount (g). Also 15 minutes after the start of the first input of the test liquid, the same operation as in the first input was performed, and the total of the first and second inputs was taken as the total liquid backflow amount.

It can be said that the smaller the liquid backflow amount is, the more preferable the absorbent article is.

$$\text{Liquid backflow amount (g)} = \text{Wf} - \text{We}$$

<Production of Water-Absorbent Resin>

(Example 1)

A 2-L round-bottomed cylindrical separable flask having an inner diameter of 11 cm and equipped with a reflux condenser, a dropping funnel, a nitrogen gas introduction tube, and a stirring blade with double 4-winged inclined paddles having a blade diameter of 5 cm as a stirrer was prepared. To this flask, 293 g of n-heptane as the hydrocarbon dispersion medium was charged, 0.736 g of a maleic anhydride-modified ethylene-propylene copolymer (Mitsui Chemicals, Inc., Hi-WAX 1105A) as the polymeric dispersant was added, the temperature was raised to 80°C with stirring at a rotation speed of the stirrer of 300 rpm to dissolve the dispersant, and then the dispersant solution formed was cooled to 50°C.

On the other hand, 92.0 g (1.03 mol) of a 80.5-mass% aqueous acrylic acid solution as the ethylenically unsaturated monomers was put in a beaker having an internal volume of 300 ml, 147.5 g of a 20.9-mass% aqueous sodium hydroxide solution was added dropwise with cooling from the outside to perform neutralization of 75 mol%, and then 0.092 g of hydroxyethyl cellulose (Sumitomo Seika Chemicals Company, Limited, HEC AW-15F) as the thickener, 0.0736 g (0.272 mmol) of potassium persulfate as the radical polymerization agent, and 0.00276 g (0.016 mmol) of ethylene glycol diglycidyl ether as the internal crosslinking agent were added thereto and dissolved, thereby preparing a first-step aqueous monomer solution.

The prepared first-step aqueous monomer solution was added to the dispersant solution in the separable flask and stirred for 10 minutes to obtain a suspension. Next, a surfactant solution obtained by heating and dissolving 0.736 g of sucrose stearate (HLB: 3, Mitsubishi-Chemical Foods Corporation, RYOTO Sugar Ester S-370) as the surfactant in 6.62 g of n-heptane was added to the suspension, and the inside of the system was sufficiently purged with nitrogen while stirring at a rotation speed of a stirrer of 550 rpm. Thereafter, the flask was immersed in a water bath at 70°C to raise the temperature, and polymerization was performed for 60 minutes to provide a first-step polymerization slurry.

Next, 128.8 g (1.43 mol) of a 80.5-mass% aqueous acrylic acid solution as the ethylenically unsaturated monomers was placed in another beaker having an internal volume of 500 ml, and 160.7 g of a 27-mass% aqueous sodium hydroxide solution was added dropwise thereto with cooling from the outside to perform neutralization of 75 mol%. Thereafter, 0.090 g (0.334 mmol) of potassium persulfate as the radical polymerization initiator and 0.0116 g (0.067 mmol) of ethylene glycol diglycidyl ether as the internal crosslinking agent were added and dissolved, thereby preparing a second-step aqueous monomer solution.

The inside of the system in the separable flask was cooled to 25°C with stirring at a rotation speed of the stirrer of 1,000 rpm. Then, the whole amount of the second-step aqueous monomer solution was added to the first-step polymerization slurry in the separable flask, and the inside of the system was purged with nitrogen for 30 minutes. Thereafter, the flask was immersed in a water bath at 70°C again to raise the temperature, and a polymerization reaction was performed for 60 minutes.

To the reaction solution containing the hydrogel-like polymer after the second-step polymerization, 0.491 g of a 45-mass% aqueous pentasodium diethylenetriaminepentaacetate solution was added under stirring. Thereafter, the flask was immersed in an oil bath set at 125°C, and 258.0 g of water was removed to the outside of the system while refluxing n-heptane by azeotropic distillation of n-heptane and water. Thereafter, 2.21 g (0.25 mmol) of a 2-mass% aqueous ethylene glycol diglycidyl ether solution as the post-crosslinking agent was added to the flask, and the mixture was held at 83°C for 2 hours.

Thereafter, n-heptane was evaporated at 125°C and dried to obtain polymer particles (dry product). The polymer particles were passed through a sieve with an aperture size of 850 μm, and 0.5 mass% of amorphous silica (Oriental Silicas Corporation, Tokusil NP-S, hydrophilic) with respect to the mass of the polymer particles was mixed with the polymer particles to provide 230.9 g of a water-absorbent resin containing amorphous silica. The median particle diameter of the water-absorbent resin was 385 μm. The 5-minute non-pressurized DW was 67 ml/g.

(Example 2)

Except that the amount of water to be removed to the outside of the system by azeotropic distillation was changed to 266.6 g, and the amount of the aqueous ethylene glycol diglycidyl ether solution as the post-crosslinking agent was changed to 4.42 g (0.507 mmol), 224.6 g of a water-absorbent resin was obtained in the same manner as in Example 1. The median particle diameter of the water-absorbent resin was 341 μm. The 5-minute non-pressurized DW was 54 ml/g.

(Example 3)

Except that the amount of water to be removed to the outside of the system by azeotropic distillation was changed to 277.9 g, and the amount of the aqueous ethylene glycol diglycidyl ether solution as the post-crosslinking agent was changed to 8.83 g (1.014 mmol), 229.1 g of a water-absorbent resin was obtained in the same manner as in Example 1. The median particle diameter of the water-absorbent resin was 391 μm. The 5-minute non-pressurized DW was 56 ml/g.

(Example 4)

Except that the amount of water to be removed to the outside of the system by azeotropic distillation was changed to 262.9 g, and the amount of the aqueous ethylene glycol diglycidyl ether solution as the post-crosslinking agent was changed to 5.52 g (0.634 mmol), 228.0 g of a water-absorbent resin was obtained in the same manner as in Example 1. The median particle diameter of the water-absorbent resin was 393 μm. The 5-minute non-pressurized DW was 64 ml/g.

(Example 5)

Except that the addition amount of n-heptane as the hydrocarbon dispersion medium and the addition amount of the maleic anhydride-modified ethylene-propylene copolymer as the polymeric dispersant were changed to 294 g and 0.644 g, respectively, in the preparation of the separable flask, hydroxyl ethyl cellulose as the thickener was not added and the amount of ethylene glycol diglycidyl ether as the internal crosslinking agent was changed to 0.0046 g (0.026 mmol) in the preparation of the first-step aqueous monomer solution, the amount of n-heptane was changed to 5.80 g, the addition amount of sucrose stearate as the surfactant was changed to 0.644 g, and the rotation speed of the stirrer was changed to 500 rpm in the step of adding the heat-dissolved surfactant solution into the separable flask, ethylene glycol diglycidyl ether as the internal crosslinking agent was not added in the preparation of the second-step aqueous monomer solution, and the amount of water to be removed to the outside of the system by azeotropic distillation was changed to 251.2 g, 219.2 g of a water-absorbent resin was obtained in the same manner as in Example 1. The median particle diameter of the water-absorbent resin was 327 μm. The 5-minute non-pressurized DW was 62.4 ml/g.

(Example 6)

Except that the addition amount of n-heptane as the hydrocarbon dispersion medium and the addition amount of the maleic anhydride-modified ethylene-propylene copolymer as the polymeric dispersant were changed to 294 g and 0.644 g, respectively, in the preparation of the separable flask, hydroxyl ethyl cellulose as the thickener was not added and ethylene glycol diglycidyl ether as the internal crosslinking agent was not added in the preparation of the first-step aqueous monomer solution, the amount of n-heptane was changed to 5.80 g, the addition amount of sucrose stearate as the surfactant was changed to 0.644 g, and the rotation speed of the stirrer was changed to 500 rpm in the step of adding the heat-dissolved surfactant solution into the separable flask, the amount of water to be removed to the outside of the system by azeotropic distillation was changed to 265.7 g, and the amount of the aqueous ethylene glycol diglycidyl ether solution as the post-crosslinking agent was changed to 5.52 g (0.634 mmol), 214.4 g of a water-absorbent resin was obtained in the same manner as in Example 1. The median particle diameter of the water-absorbent resin was 340 μm. The 5-minute non-pressurized DW was 46.0 ml/g.

(Comparative Example 1)

Except that the amount of water to be removed to the outside of the system by azeotropic distillation was changed to 286.8 g, and the amount of the aqueous ethylene glycol diglycidyl ether solution as the post-crosslinking agent was changed to 13.25 g (1.521 mmol), 227.6 g of a water-absorbent resin was obtained in the same manner as in Example 1. The median particle diameter of the water-absorbent resin was 385 μm. The 5-minute non-pressurized DW was 41 ml/g.

(Comparative Example 2)

Except that the amount of water to be removed to the outside of the system by azeotropic distillation was changed to 275.7 g, 228.9 g of a water-absorbent resin was obtained in the same manner as in Comparative Example 2. The median particle diameter of the water-absorbent resin was 354 μm. The 5-minute non-pressurized DW was 53 ml/g.

(Comparative Example 3)

Except that 0.092 g (0.339 mmol) of 2,2'-azobis(2-amidinopropane) dihydrochloride as the radical initiator was added and the amount of potassium persulfate as the radical polymerization agent was changed to 0.018 g (0.068 mmol) in the preparation of the first-step aqueous monomer solution, the amount of ethylene glycol diglycidyl ether as the internal crosslinking agent was changed to 0.0046 g (0.026 mmol), 0.129 g (0.475 mmol) of 2,2'-azobis(2-amidinopropane) dihydrochloride as the radical polymerization agent was added and the amount of potassium persulfate as the radical polymerization agent was changed to 0.026 g (0.095 mmol) in the preparation of the second-step aqueous monomer solution, the amount of water removed to the outside of the system by azeotropic distillation was changed to 234.2 g, an aqueous ethylene glycol diglycidyl ether solution as the post-crosslinking agent was not added, and the mixing amount of amorphous silica with respect to polymer particles (dry product) was changed to 0.2 mass%, 227.8 g of a water-absorbent resin was obtained in the same manner as in Example 1. The median particle diameter of the water-absorbent resin was 382 μm. The 5-minute non-pressurized DW was 0.4 ml/g.

(Comparative Example 4)

A water-absorbent resin collected from an absorbent body of an adult disposable diaper "Refré Slim Pull-on Pants M size" (purchased in 2020) manufactured by LiveDo Corporation was obtained. Since the water-absorbent resin in the absorbent body was mixed with pulp, the pulp was removed as completely as possible with a jet of air. The median particle diameter of the water-absorbent resin collected was 384 μm. The 5-minute non-pressurized DW was 35 ml/g.

[Table 1]

| Table 1 | Water-absorbent resin | | | Absorbent article | | | | |
| | Repeated water absorption amount in inclined state | Physiological saline retention | Water absorption speed of physiological saline | Leakage test | | Backflow test | | |
| | | | | Number of times of input of liquid until occurrence of leakage | Absorption amount of liquid until occurrence of leakage | First backflow amount | Second backflow amount | Total backflow amount |
| | [g] | [g/g] | [sec] | [times] | [g] | [g] | [g] | [g] |
| Example 1 | 40 | 48 | 44 | 4 | 294 | 4 | 68 | 72 |
| Example 2 | 40 | 50 | 37 | 4 | 285 | 11 | 66 | 77 |
| Example 3 | 38 | 48 | 44 | 4 | 279 | 9 | 67 | 76 |
| Example 4 | 38 | 45 | 41 | 4 | 271 | 9 | 67 | 76 |
| Example 5 | 40 | 55 | 35 | 5 | 350 | 3 | 64 | 67 |
| Example 6 | 40 | 63 | 49 | 4 | 320 | 1 | 60 | 61 |
| Comparative Example 1 | 34 | 49 | 47 | 3 | 229 | 13 | 61 | 74 |
| Comparative Example 2 | 30 | 35 | 37 | 3 | 239 | 14 | 73 | 87 |
| Comparative Example 3 | 18 | 93 | 79 | 2 | 153 | 16 | 58 | 74 |
| Comparative Example 4 | 26 | 49 | 35 | 3 | 218 | 7 | 72 | 79 |

DESCRIPTION OF REFERENCE SIGNS

1: Burette unit
3: Clamp
5: Conduit
10a: Water-absorbent resin
11: Stand
13: Measurement stage
13a: Through hole
15: Nylon mesh sheet
21: Burette tube
22: Cock
23: Rubber stopper
24: Cock
25: Air introduction tube
40: Supporting plate
41: Stand
42: Dropping funnel
43: Balance
44: Metal tray
50: Saline
51: Test liquid
100: Absorbent article
$S_0$: Horizontal plane
$S_1$: Inclined surface

**Claims**

1. A water-absorbent resin having a repeated water absorption amount in an inclined state of 35 g or more as measured by a measurement method below:

   <Method for measuring repeated water absorption amount in inclined state>
   In a petri dish made of plastic and having a circular shape, an inner diameter of 8.5 cm, and a height of 1.7 cm, 1.0 g of the water-absorbent resin is uniformly spread; subsequently, 30 g of physiological saline is supplied at once on the water-absorbent resin, and immediately the petri dish is shaken 10 times from side to side and then allowed to stand for 3 minutes to form a swollen gel layer containing the water-absorbent resin having absorbed the physiological saline; separately, a device is prepared including an acrylic plate having a main surface having a flat shape fixed in such a manner that the main surface is at an angle of 30° to a horizontal plane; on an upper surface of the swollen gel layer, an air through non-woven fabric having a weight per unit area of 21 $g/m^2$ and cut into a same size as an inside of the petri dish is placed, and the petri dish is fixed to the acrylic plate with tape to avoid slipping off; from a vertical height of approximately 1 cm at a center of the swollen gel layer in the petri dish, 5 g of the physiological saline is poured for 5 seconds using a macropipette; the pouring is repeated 8 times at 1 minute intervals from the start of the pouring of the physiological saline; and an amount of liquid leaked from the petri dish is measured and subtracted from a poured amount of 40 g of the physiological saline, and a calculated mass of the physiological saline retained by the swollen gel layer is regarded as the repeated water absorption amount of the water-absorbent resin.

2. The water-absorbent resin according to claim 1, having a median particle diameter of 200 $\mu$m or more and 700 $\mu$m or less.

3. The water-absorbent resin according to claim 1 or 2, having a water absorption speed of the physiological saline of 20 seconds or more and 70 seconds or less.

4. An absorbent body comprising the water-absorbent resin according to any one of claims 1 to 3.

**5.** An absorbent article comprising the absorbent body according to claim 4.

FIG. 1

FIG. 2

## INTERNATIONAL SEARCH REPORT

| | International application No. |
|---|---|
| | **PCT/JP2022/015354** |

**A. CLASSIFICATION OF SUBJECT MATTER**

*C08F 2/00*(2006.01)i; *A61F 13/53*(2006.01)i; *B01J 20/26*(2006.01)i; *C08F 120/06*(2006.01)i
FI: C08F2/00; A61F13/53 300; B01J20/26 D; C08F120/06

According to International Patent Classification (IPC) or to both national classification and IPC

**B. FIELDS SEARCHED**

Minimum documentation searched (classification system followed by classification symbols)

C08F2/00; A61F13/53; B01J20/26; C08F120/06

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO 2020/218160 A1 (SUMITOMO SEIKA CHEMICALS CO., LTD.) 29 October 2020 (2020-10-29) <br> claims, examples | 1-5 |
| X | WO 2020/184398 A1 (SUMITOMO SEIKA CHEMICALS CO., LTD.) 17 September 2020 (2020-09-17) <br> claims, examples | 1-5 |
| X | WO 2020/184393 A1 (SUMITOMO SEIKA CHEMICALS CO., LTD.) 17 September 2020 (2020-09-17) <br> claims, examples | 1-5 |

☐ Further documents are listed in the continuation of Box C.    ☑ See patent family annex.

| | |
|---|---|
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **02 May 2022** | **17 May 2022** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

INTERNATIONAL SEARCH REPORT
Information on patent family members

International application No.

**PCT/JP2022/015354**

| Patent document cited in search report | | | Publication date (day/month/year) | Patent family member(s) | Publication date (day/month/year) |
|---|---|---|---|---|---|
| WO | 2020/218160 | A1 | 29 October 2020 | (Family: none) | |
| WO | 2020/184398 | A1 | 17 September 2020 | (Family: none) | |
| WO | 2020/184393 | A1 | 17 September 2020 | (Family: none) | |

Form PCT/ISA/210 (patent family annex) (January 2015)

**REFERENCES CITED IN THE DESCRIPTION**

**Patent documents cited in the description**

- JP H3227301 A